# EUROPEAN PATENT SPECIFICATION

(11) **EP 4 447 832 B1**
(45) Date of publication and mention of the grant of the patent: **17.06.2026**
(21) Application number: 22854220.5
(22) Date of filing: 16.12.2022
(51) Int. Cl.: A61B 17/80, A61F 2/30, A61F 2/44, A61F 2/46

(54) **IMPLANT ASSEMBLY WITH EXPANDABLE IMPLANT, PLATE AND INSTALLATION TOOL**
IMPLANTATANORDNUNG MIT EXPANDIERBAREM IMPLANTAT, PLATTE UND INSTALLATIONSWERKZEUG
ENSEMBLE D'IMPLANT AVEC IMPLANT EXPANSIBLE, PLAQUE ET OUTIL D'INSTALLATION

(30) Priority: 17.12.2021 US 202117555011
(43) Date of publication of application: 23.10.2024
(73) Proprietor: Life Spine, Inc., Huntley IL 60142 (US)
(72) Inventor: PREDICK, Daniel, Wheat Ridge, Colorado 80215 (US); ZAKELJ, Paul Christopher, Chicago, Illinois 60618 (US)
(74) Representative: V.O.
(86) International application number: PCT/US2022/053230
(87) International publication number: WO 2023/114506

(56) References cited:
- WO-A1-2017/106614
- WO-A1-2019/126213
- WO-A1-2021/030645
- FR-A1- 2 894 130
- US-A1- 2020 129 307
- US-A1- 2021 045 891
- US-A1- 2021 322 181

## Description

### CROSS-REFERENCE TO RELATED PATENT APPLICATIONS

The present application is a continuation of and claims priority to U.S. Application No. 17/555,011, filed December 17, 2021.

### BACKGROUND

The present disclosure relates to implant assemblies such as expandable implants and devices, including spinal interbody and intravertebral body devices, and vertebral interbody and intravertebral devices that are expandable after placement thereof. The implants disclosed herein may be usable in spinal applications as well as other non-spinal applications.

Fusion cages, as well as other types of implants, bodies and/or devices, are frequently utilized in spinal surgery inside a vertebra (intravertebral) and/or between vertebrae of a patient (interbody), or adjacent other bone bodies. With interbody devices, one or more such spinal bodies are placed between vertebrae to provide support and promote fusion between adjacent vertebrae where such is necessary due to disease, injury, general deterioration or congenital problem. With intravertebral devices, one or more spinal bodies are placed within a vertebra. Spinal devices, such as fusion cages and/or the like, are inserted into a spinal space either anteriorly, posteriorly, laterally or posteriolaterally.

Certain implants are expandable and positionable by a user, such as a surgeon. WO 2017/106614 A1, US 2021/045891 A1, WO 2021/030645 A1 and WO 2019/126213 A1 show examples of expandable interbody spinal fusion devices.

Expandable interbody devices allow the device to be initially smaller than traditional non-expandable (static) interbody devices such that expandable interbody devices may be more easily inserted or implanted into the vertebral space. Moreover, expandable devices allow the surgeon to set the amount of expansion necessary for the particular patient rather than the static device dictating the spacing.

### SUMMARY

The present invention relates to an implant assembly as claimed hereafter. Preferred embodiments of the invention are set forth in the dependent claims. One embodiment relates to an implant assembly including an expandable implant, the expandable implant having a base member and an adjustable member movable relative to the base member such that the implant is movable between a collapsed configuration and an expanded configuration. The base member includes an attachment port. The implant assembly further includes a plate configured to be coupled to the expandable implant, a coupling fastener configured to couple the plate to the expandable implant, and at least one bone screw configured to extend through the plate and into an adjacent portion of bone. The attachment port is configured to receive an installation tool during positioning of the expandable implant and to receive the coupling fastener to couple the plate to the expandable implant.

Another embodiment relates to a method (not claimed) of installing an implant assembly, including coupling a tool to an attachment port of an expandable implant; positioning the expandable implant in a desired location using the tool; expanding the expandable implant to a desired height; coupling a plate to the expandable implant by securing a coupling fastener to the plate and the expandable implant; and installing a plurality of bone screws through the plate and into adjacent portions of bone. The coupling fastener is received by the attachment port of the expandable implant to couple the plate to the expandable implant.

Another embodiment relates to an implant assembly including at least one installation tool and an expandable implant, the expandable implant including a base member and an adjustable member movable relative to the base member such that the expandable implant is movable between a collapsed configuration and an expanded configuration. The expandable implant includes an attachment port. The assembly further includes a plate configured to be coupled to expandable implant via the attachment port; a coupling fastener configured to couple the plate to the expandable implant; and at least one bone screw configured to extend through the plate and into an adjacent portion of bone. The attachment port is configured to couple with the at least one installation tool during positioning of the expandable implant and to couple with the coupling fastener to secure the plate to the expandable implant.

This summary is illustrative only and is not intended to be in any way limiting. Other aspects, inventive features, and advantages of the devices or processes described herein will become apparent in the detailed description set forth herein, taken in conjunction with the accompanying figures, wherein like reference numerals refer to like elements.

### BRIEF DESCRIPTION OF THE FIGURES

FIG. 1 is perspective view of an expandable implant in a collapsed position according to one embodiment.
FIG. 2 is a perspective view of the implant of FIG. 1 in an expanded position according to one embodiment.
FIG. 3 is another perspective view of the implant of FIG. 1 in an expanded position according to one embodiment.
FIG. 4 is another perspective view of the implant of FIG. 1 in an expanded position according to one embodiment.
FIG. 5 is a top view of the implant of FIG. 1 according to one embodiment.
FIG. 6 is a bottom perspective view of the implant of FIG. 1 according to one embodiment.
FIG. 7 is an exploded view of the implant of FIG. 1 according to one embodiment.
FIG. 8 is a side cross-sectional view of the implant of FIG. 1 in a collapsed position according to one embodiment.
FIG. 9 is a side cross-sectional view of the implant of FIG. 1 in an expanded position according to one embodiment.
FIG. 10 is a perspective view of an implant assembly using the implant of FIG. 1 according to one embodiment.
FIG. 11 is a side view of the implant assembly of FIG. 10 according to one embodiment.
FIG. 12 is a perspective view of a plate usable as part of the implant assembly of FIG. 10 according to one embodiment.
FIG. 13 is a partial exploded view of the plate of FIG. 12 and fasteners usable as part of the implant assembly of FIG. 10 according to one embodiment.
FIG. 14 is a perspective view of the plate and fasteners of FIG. 13 in an assembled configuration according to one embodiment.
FIG. 15 is a side view of the plate of FIG. 12 according to one embodiment.
FIG. 16 is another side view of the plate of FIG. 12 according to one embodiment.
FIG. 17 is a perspective view of an implant assembly using the implant of FIG. 1 according to another embodiment.
FIG. 18 is a side view of the implant assembly of FIG. 17 according to one embodiment.
FIG. 19 is a perspective view of a plate usable as part of the implant assembly of FIG. 17 according to one embodiment.
FIG. 20 is a partial exploded view of the plate of FIG. 19 and fasteners usable as part of the implant assembly of FIG. 17 according to one embodiment.
FIG. 21 is a perspective view of the plate and fasteners of FIG. 20 in an assembled configuration according to one embodiment.
FIG. 22 is a side view of the plate of FIG. 19 according to one embodiment.
FIG. 23 is another side view of the plate of FIG. 19 according to one embodiment.

### DETAILED DESCRIPTION

Before turning to the figures, which illustrate certain exemplary embodiments in detail, it should be understood that the present disclosure is not limited to the details or methodology set forth in the description or illustrated in the figures. It should also be understood that the terminology used herein is for the purpose of description only and should not be regarded as limiting.

The present disclosure relates to expandable and/or dynamic implants, including, but not limited to, interbody (between adjacent vertebrae), intravertebral-body (inside the vertebrae) and/or spinal stabilization devices that may or may not be used as interbody fusion cages or devices, interbody/intravertebral bodies/body stabilization devices and/or the like (e.g., spinal device(s)) for providing support, stabilization and/or promoting bone growth between or inside vertebrae or other portions of bone that have been destabilized or otherwise due to injury, illness and/or the like. Particularly, the present disclosure provides various versions of dynamic (expandable and/or expandable and retractable) interbody/intravertebral body devices that are usable in a spinal column or other areas of a human.

Various embodiments disclosed herein are directed to expandable implants that are implantable between adjacent bodies of bone. For example, the implant may be implanted or inserted into a human spine adjacent upper and lower vertebrae of the spine. According to various exemplary embodiments, the components of the implants disclosed herein may be made of any suitable material(s), including a variety of metals, plastics, composites, or other suitable bio-compatible materials. In some embodiments, one or more components of the implants disclosed herein may be made of the same material, while in other embodiments, different materials may be used for different components of the various implants.

Some embodiments disclosed herein relate to an implant assembly including an expandable implant, a plate, and one or more bone screws. The expandable implant is configured to be positioned in a desired location and expanded to a desired degree of expansion. The plate may be coupled to the expandable implant (either prior to or after expansion), and one or more bone screws passed through the plate and into adjacent portions of bone (e.g., to one or both of an upper vertebral body and a lower vertebral body) to secure the plate and the implant assembly in a desired position. In some embodiments, the expandable implant includes an attachment port. The attachment port may receive a tool usable to position the expandable implant in a desired position. The attachment port may receive the tool in a threaded manner. The attachment port may further receive a coupling fastener usable to couple the plate to the expandable implant. The coupling fastener may be a screw and the attachment port may receive the screw in a threaded manner. As such, the attachment port may be usable to couple with (e.g., threadingly engage) both the installation tool and the coupling fastener.

One or more alignment apertures may be provided on the expandable implant. In one embodiment, two alignment apertures are provided adjacent the attachment port for coupling with an installation tool, which may be the same or different tool from the tool that couples to the attachment port. The alignment apertures may be anti-rotation holes provided on opposite sides of the attachment port such that the anti-rotation holes are usable (e.g., through engagement with a tool) to resist rotation of the expandable implant.

The expandable implant may include a base member, an adjustable member adjustably coupled to the base member and movable between a first, collapsed position, and a second, expanded position, and a control shaft rotatably received by the base member, where rotation of the control shaft cause relative movement of the adjustable member relative to the base member. At least one control member is received on the control shaft and by the control channel, and rotation of the control shaft causes the control member to translate along the control shaft and along the control channel. In one embodiment, first and second control members are received on first and second control threads of the control shaft. The first control thread may have a larger diameter than the second control thread, and the first control member may have a larger threaded bore that the second control member, such that both control members may be installed over an end of the control shaft (e.g., by passing the first control member (having a larger diameter) over the second control thread (having a smaller diameter) in a non-threaded manner).

In some embodiments, the expandable implant is usable with any of a number of plates. The plates may be modular in that any one of a plurality of plates may be interchangeably used to couple with the expandable implant. The plates may take different sizes, and may be configured to receive bone screws to secure the plates to adjacent portions of bone. The plates may further include projections, or spikes, to engage bone and/or facilitate preventing rotation of the plate and the expandable implant. Different plates may differ in the number of bone screws usable with the plate, the position of the bone screw bores that receive the bone screws, the number and/or position of the projections, the size and/or shape of the plate, etc. In one embodiment, each plate is a single, integral piece (e.g., a monolithic component). Providing an integral plate may provide increased stability relative to multi-piece plates. In other embodiments, the plate may be attached to the expandable implant prior to expansion of the implant.

The expandable implant and one or more plates may be provided as a kit. Providing different modular plates provides a user (e.g. a surgeon) with different options to select from depending on a particular application. The kit may include an installation tool usable to position and/or expand the expandable implant. The same or different tool may be usable to secure the plate to the expandable implant.

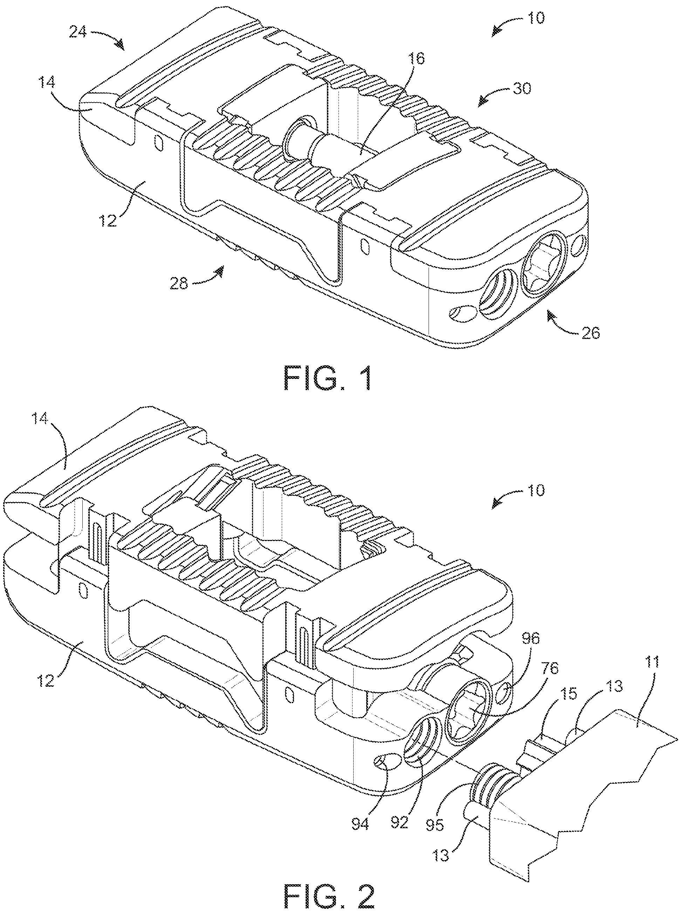

Referring now to FIGS. 1-9, an expandable implant 10 is shown according to an exemplary embodiment. Implant 10 is usable, for example, between and/or within vertebral bodies of the spine. It should be understood that implant 10 may in some embodiments be usable in other portions of the body in addition to the spine, and all such applications are to be understood to be within the scope of the present disclosure.

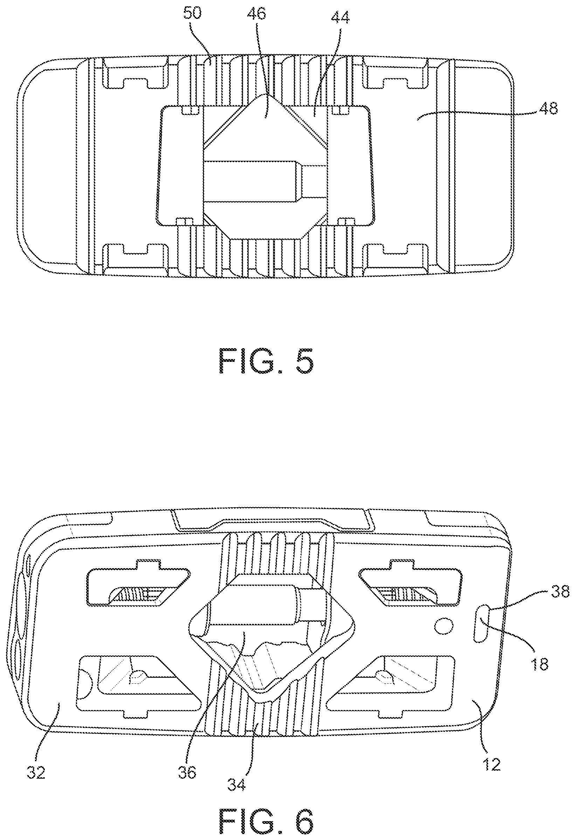

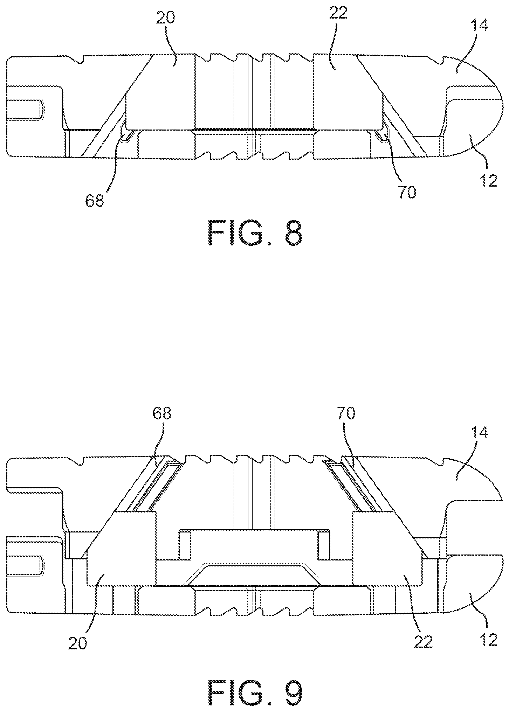

According to an exemplary embodiment, implant 10 includes a base member 12 (e.g., a first or lower member, support, etc.) and an adjustable member 14 (e.g., a second or upper member, support, etc.) adjustably coupled to the base member 12. The implant has a front or first end 24, a rear or second end 26, a first side 28, and a second side 30. A control shaft 16 is received by the base member 12 and is retained by a retention member 18 (see FIG. 6) passing through a portion of the base member 12. A first control member 20 and a second control member 22 are received on the control shaft 16 and are movable along the control shaft 16 to adjust a position of the adjustable member 14 between a collapsed position, as shown in FIGS. 1 and 8, and an expanded position, as shown in FIGS. 2 and 9.

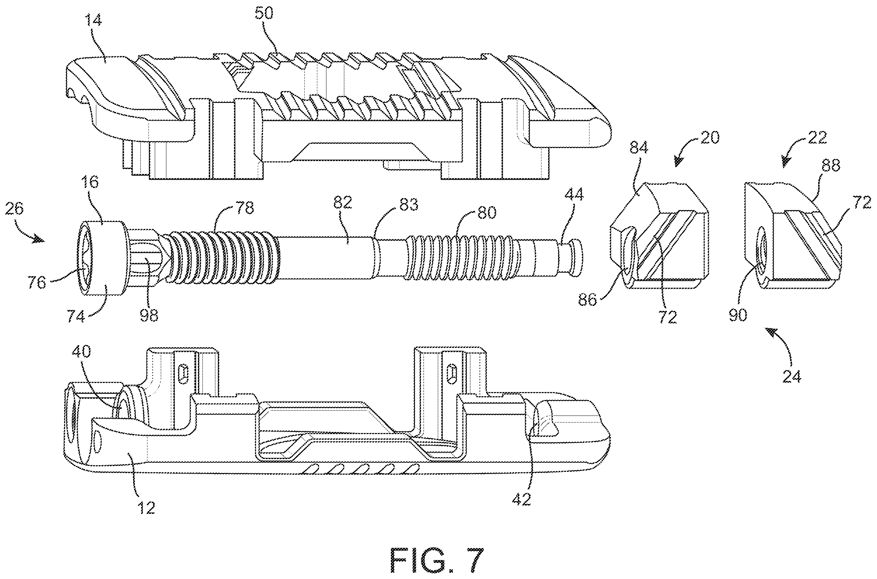

In one embodiment, the base member 12 includes a central cavity 36 disposed between the first end 24 and the second end 26 (see FIG. 6). The base member 12 further includes a bottom surface 32 having ridges or projections 34 formed by corresponding grooves. The projections 34 are configured to engage adjacent portions of bone. An aperture 38 extends through bottom surface 32 and is configured to receive the retention member 18 (e.g., in a press fit or other manner). As shown in FIG. 7, at second end 26, the base member 12 includes a control bore 40 configured to receive a first portion of the control shaft 16. At first end 24, the base member 12 includes a control bore 42 configured to receive a second portion of the control shaft 16. Shaft 16 includes a channel 44 (e.g., a groove, recess, etc.) configured to receive retention member 18. In one embodiment, channel 44 and retention member 18 are configured to enable rotation of shaft 16 relative to base member 12, but prevent translation of shaft 16 relative to base member 12.

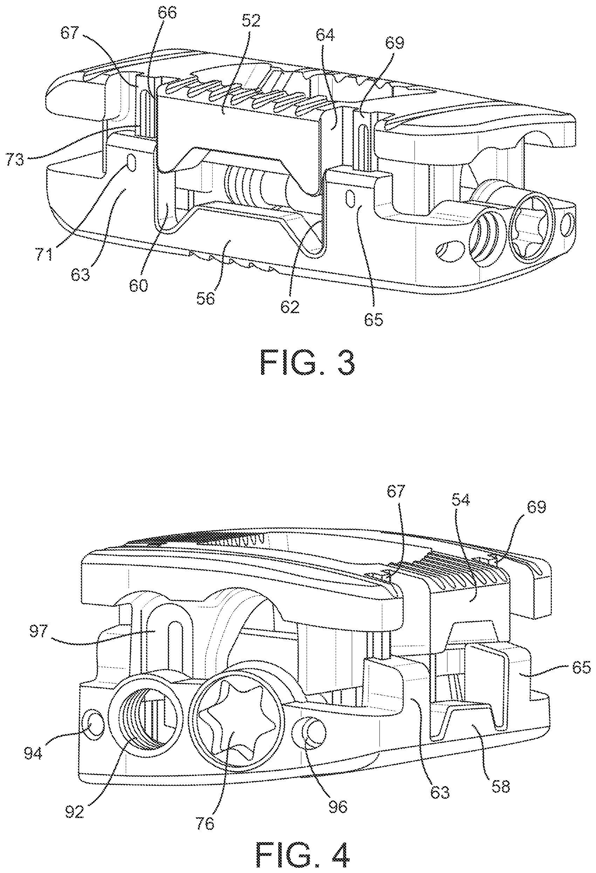

In one embodiment, the adjustable member 14 includes a central recess or cavity 46 positioned between first end 24 and the second end 26 (see FIG. 5). The adjustable member 14 further includes a top surface 48 having ridges or projections 50 formed by corresponding grooves, and first and second side portions 52, 54 (see FIGS. 3-4). In some embodiments, the first and second side portions 52, 54 have shapes generally corresponding to the shapes of the first and second side portions 56, 58 of base member 12. In other embodiments, the first and second side portions 52, 54 have shapes differing from the shapes of the first and second side portions 56, 58 of the base member 12. In one embodiment, side portions 56, 58 define upstanding sidewalls 60, 62 (see FIG. 3) configured to slidingly engage corresponding sidewalls 64, 66 on first and second side portions 52, 54. In further embodiments, base member 12 includes alignment structures 63, 65 that are configured to engage (e.g., slidingly engage, etc.) alignment structures 67, 69 on adjustable member 14. For example, alignment structures 63, 65 may include one or more rails, channels, etc. that are received in and/or receive corresponding rails, channels, etc. on alignment structures 67, 69. Furthermore, alignment structures 63, 65, and 67, 69 may utilize a pin and slot or other arrangement to limit expansion of implant 10. For example, alignment structures 63, 65 may include pins 71 (e.g., projections, etc.) that are received in recesses 73 (e.g., channels, slots, etc.) in alignment structures 67, 69, such that the maximum travel of pins 71 within recesses 73 limits the maximum expansion of implant 10. The pins and slots may be provided on one or both sides of implant 10.

Referring to FIGS. 8-9, in one embodiment, the adjustable member 14 includes one or more control channels, such as a first control channel 68 and a second control channel 70. The first control channel 68 receives the first control member 20, and the second control channel 70 receives the second control member 22. In some embodiments, the control members 20, 22 are received in the control channels 68, 70 in a sliding manner such that the control members 20, 22 are able to translate within the control channels 68, 70. In further embodiments, each control channel has a shape such that the control channel partially surrounds the control member and at least partially corresponds in shape to the control member. In some embodiments, each control member 20, 22 includes one or more projections 72 (see FIG. 7) that are received within control channels 68, 70. Projections 72 and control channels 68, 70 may in some embodiments prevent relative rotation between control members 20, 22 and adjustable member 14.

Referring back to FIG. 7, the control shaft 16 includes a head portion 74, a tool port 76 disposed within the head portion 74, and channel 44 located at an end opposite the head portion 74. In some embodiments, the control shaft 16 further includes a first control thread 78 (e.g., a first threaded portion, etc.) and a second control thread 80 (e.g., a second threaded portion, etc.). A non-threaded portion 82 may be located between the first control thread 78 and the second control thread 80. As shown in FIG. 7, the non-threaded portion 82 may include a "step" or transition area 83 between a larger diameter portion and a smaller diameter portion of control shaft 16.

The first control member 20 includes one or more flat portions 84, and a first internal thread 86. The second control member 22 includes one or more flat portions 88, and a second internal thread 90. First control member 20 threadingly engages first control thread 78, and second control member 22 threadingly engages second control thread 80. As shaft 16 rotates, first control member 20 translates along first control thread 78 and second control member 22 translates along second control thread 80. In some embodiments, first control thread 78 is opposite-threaded from second control thread 80. In one embodiment, first control thread 78 is a larger diameter than second control thread 80, and first internal thread 86 of first control member 20 has a larger diameter than second internal thread 90 of second control member 22, such that first control member 20 can be passed over second control thread 80 (without threaded engagement) and into threaded engagement with first control thread 78.

According to one embodiment, the control shaft 16 is received by the base member 12 such that the retention groove 44 is positioned at first end 24 and the head portion 90 is positioned at second end 26. The control shaft 16 is rotatable within the base member 12, and the retention member 18 extends through the base member 12 at first end 24 and into the retention groove 44 of the control shaft 16 to enable rotation of the control shaft 16 while inhibiting translation of the control shaft 16 relative to the base member 12. The first control member 20 is received on the first control thread 78 of the control shaft 16, and the second control member 22 is received on the second control thread 80 of the control shaft 16. As noted above, to facilitate assembly of implant 10, the larger diameter of first internal thread 86 enables passing of the first control member 20 over the second control thread 80 in a non-threaded manner.

In one embodiment, the first control thread 78 and the second control thread 80 are threaded in opposite manners (e.g., left-handed and right-handed), such that upon rotation of the control shaft 16, the control members 20, 22 move in opposite directions along the control shaft 16. For example, the control shaft 16 may be configured such that rotation of the control shaft 16 in a first direction (e.g., clockwise) causes the first and second control members 20, 22 to move toward each other, and rotation of the control shaft 16 in a second direction (e.g., counter-clockwise) causes the first and second control member 20, 22 to move away from each other.

In some embodiments, the control members 20, 22 are configured to provide an expansion or contraction force (e.g., a wedging force) to adjustable member 12 depending on the direction of movement of the control members (e.g., for either expansion or contraction of the implant). For example, during expansion, control members 20, 22 may provide an upward force to adjustable member 12, while during contraction, control members may provide a downward force to adjustable member 12. This may provide enhanced control of adjustable member 12 relative to implants that may provide, for example, only an upward force or a force in only a single direction.

As the control members 20, 22 move along the control shaft 16, the control members 20, 22 further move within the control channels 68, 70, thereby causing relative movement of the adjustable member 14 and the base member 12. In some embodiments, as the control members 20, 22 move away from each other along the control shaft 16, the adjustable member 14 is moved upward or downward due to the angled shape of the first and second control channels 68, 70. The rate of movement of the control members 20, 22, and therefore the adjustable member 14, can be adjusted by modifying the slope of the control channels 68, 70 relative to the control shaft 16.

In some embodiments, the first control channel 68 extends at a first angle relative to the control shaft 16, and the second control channel 70 extends at a second angle relative to the control shaft 16. The first and second angles define the rate at which first control member 20 and second control member 22 cause corresponding movement (e.g., expansion) of the opposite ends of the adjustable member 14 relative to the base member 12. In some embodiments, the first angle and second angle are approximately the same, and the control channels 68, 70 define linear paths, such that the rates of movement of the opposite ends of the adjustable member 14 are substantially the same and constant (assuming a constant rate of rotation of the control shaft 16). In other embodiments, rather than being angled toward each other in an upward direction (as shown, for example, in FIG. 9), the first and second control channels 68, 70 may extend in a parallel manner or be configured to extend upward at angles in the same general direction. In yet further embodiments, one or both of the control channels 68, 70 may define a non-linear channel. For example, second control channel 70 may define a curved path, thereby providing a changing rate of movement of one end of adjustable member 14. In further alternative embodiments, the angles of first and second control channels 68, 70 may differ from each other to provide different amounts of movement and to suit a particular application.

Providing differing configurations for the first control channel 68 and the second control channel 70 enables customization of the characteristics of the implant 10 in the second, expanded position. For example, the control channels 68, 70 may be configured such that in a fully expanded position of implant 10, one end of implant 10 (e.g., one of ends 24, 26) is expanded to a greater degree than the opposing end. Other configurations of the first and second control channels 68, 70 are possible according to various alternative embodiments.

Referring back to FIGS. 2 and 4, in one embodiment, implant 10 includes various attachment ports to facilitate using one or more tools with implant 10. For example, as shown in FIG. 4, base member 12 includes a threaded attachment port 92 offset to one side of control shaft 16, and anti-rotation ports 94, 96 on opposite sides of control shaft 16 and port 92. Attachment port 92 is configured to threadingly receive a tool such as tool 11 shown in FIG. 2 to assist in positioning implant 10 into a desired position and otherwise manipulating implant 10. While tool 11 is shown in to include a threaded shaft to engage attachment port 92, tool 11 may in other embodiments include other features and be configured to engage other portions of expandable implant 10. For example, tool 11 may include projections 13 configured to engage ports 94, 96. Ports 94, 96 are configured to enable part of a same or different tool from tool 11 to engage ports 94, 96 and inhibit rotation of implant 10 during repositioning, expansion, etc. An adjustment portion 15 may be configured to engage tool port 76 and may be provided on the same or a different tool.

Referring to FIG. 7, in some embodiments, control shaft 16 includes an access port 98 configured to provide communication (e.g., fluid or other material communication) with tool port 76. Bone graft or other material may be delivered to the interior of implant 10 via tool port 76 and access port 98. In one embodiment, at least one access port is provided. In further embodiments, two access ports 98 are provide on opposing sides of control shaft 16 between head portion 74 and first control thread 78. Access ports 98 may define axes that are angularly offset relative to the longitudinal axis of control member 16.

In use, implant 10 is positioned within a desired space (e.g., between adjacent portions of bone) while in the first, collapsed position, as shown in FIG. 1. To position implant 10, an appropriate tool such as tool 11 may be used to engage attachment port 92 and/or ports 94, 96 and manipulate implant 10 into a desired position. Once in a desired position, the same or a subsequent tool may be utilized to engage tool port 76 and rotate control shaft 16 to move adjustable member 14 to a desired degree of expansion. It should be noted that based on a particular application, the adjustable member 14 may be utilized in a fully collapsed position, a fully expanded position, or any intermediate position therebetween. Once implant 10 is properly positioned and expanded to a desired height, bone graft material may be delivered by way of, for example, tool port 76 and access port 98. The various apertures in and through the base member 12 and adjustable member 14 may in some embodiments facilitate the growth of bone or other material in and around implant 10 to further stabilize the device. As described in greater detail below, one or more plates or plates assemblies may be coupled to implant 10 and adjacent portions of bone to further secure implant 10 in a desired position.

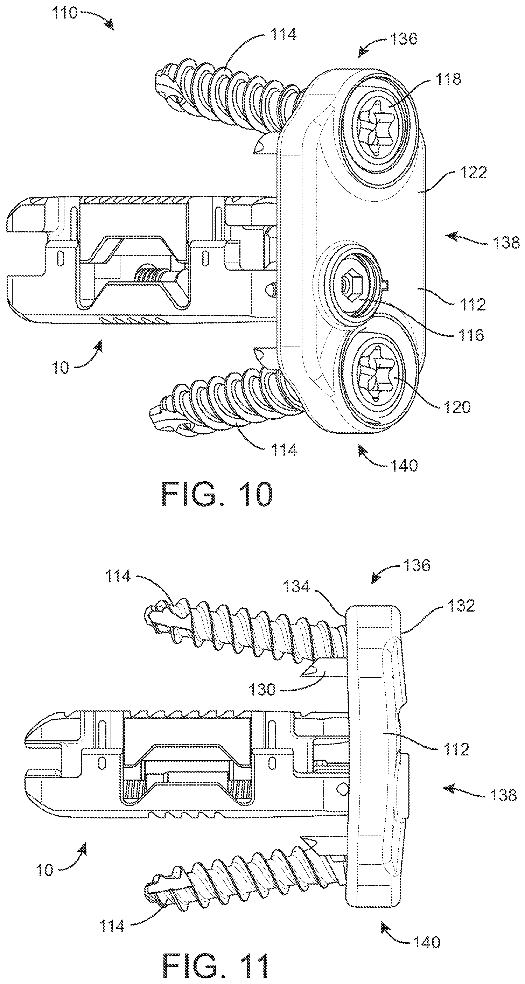

Referring now to FIGS. 10-16, an implant assembly 110 (e.g., an integrated implant, etc.) is shown according to one embodiment. Implant assembly 110 includes implant 10, a plate 112, and bone screws 114. As discussed in greater detail below, once implant 10 is secured in a desired position, plate 112 may be secured (e.g., removably coupled, etc.) to implant 10, and bone screws 114 secured to adjacent portions of bone (e.g., adjacent portions of vertebral bone).

In one embodiment, plate 112 is secured to implant 10 using a coupling screw 116. Coupling screw 116 may in one embodiment threadingly engage attachment port 92 of implant 10. In this way, attachment port 92 may be used both to receive a tool used to initially position and manipulate implant 10, and to receive a coupling screw such as coupling screw 116 used to couple plate 112 with implant 10. Bone screws 114 are configured to engage adjacent portions of bone, and are held in place using retention fasteners 118, 120. Retention fasteners 118, 120 are configured to prevent bone screws 114 from backing out once installed. In one embodiment, retention fasteners 118, 120 are set screws, and may include bottom portions 121 that may be semi-spherical and/or have a textured surface configured to provide enhanced contact with bone screws 114.

Plate 112 includes a body 122 having a top portion 136, a middle portion 138, and a bottom portion 140. Bone screw bores 126, 128 are provided in top and bottom portions 136, 140 and receive bone screws 114 and retention fasteners 118, 120 (e.g., locking screws, etc.). The distance between and positions of bone screw bores 126, 128 can be varied to suit a particular application. In one embodiment, bone screw bores 126, 128 are positioned to be adjacent corresponding upper and lower portions of bone once implant 10 is installed and expanded in a desired position and orientation.

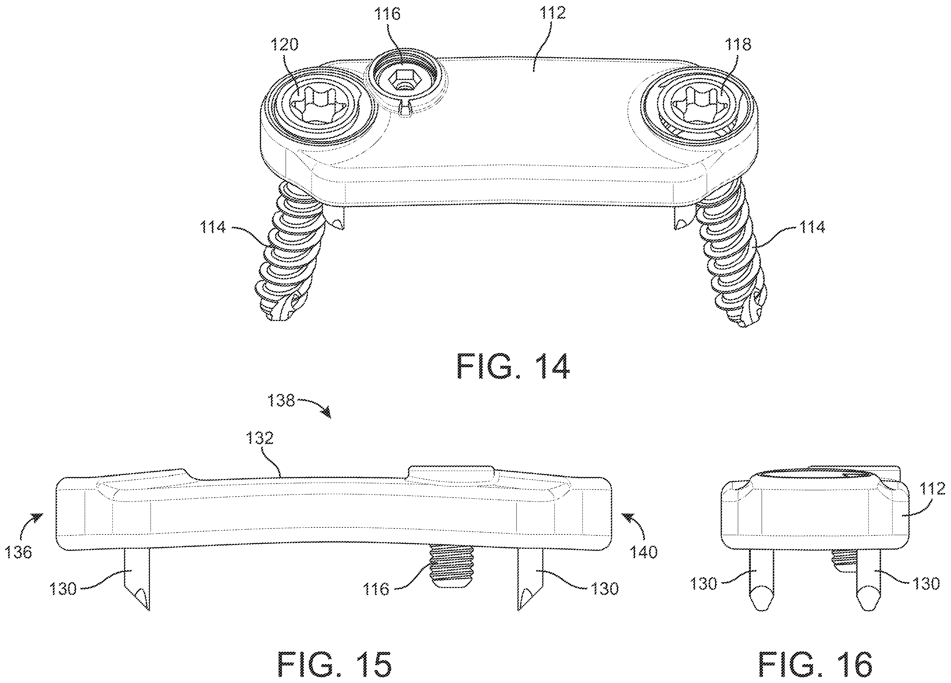

Plate 112 includes a front side 132 and a rear side 134. Bone screw bores 126, 128 and coupling screw bore 124 extend from front side 132 to rear side 134. Projections 130 extend from rear side 134 and are configured to engage adjacent portions of bone. Projections 130 may assist in preventing rotation or other movement of plate 112 during installation and/or prior to installation of bone screws 114. Projections 130 may be shaped with a pointed, sharp, and/or knife-shaped end configured to facilitate pressing or otherwise forcing projections 130 into portions of bone. As shown in Figures 15-16, in one embodiment, four projections 130 are utilized with plate 112, and projections 130 are generally positioned to define a rectangle. In alternative embodiments, more or fewer projections 130 may be used, and the projections 130 may be positioned differently than shown in FIGS. 15-16. In one embodiment, plate 112 is a single, integral piece (e.g., a monolithic component). Providing an integral plate may provide increased stability relative to multi-piece plates.

In use, a user positions implant 10 in a desired position using attachment port 92 and a suitable tool. Implant 10 may be expanded to a desired height by manipulating control shaft 16. Once implant 10 is in a desired position and at a desired degree of expansion, plate 112 may be coupled to implant 10 by threadingly coupling the coupling screw 116 to attachment port 92. Plate 112 may be positioned such that projections 130 engage adjacent portions of bone. Bone screws 114 may be inserted through plate 112 and threadingly secured to adjacent portions of bone. Retention fasteners 118, 120 may be installed to prevent backing out or loosening of bone screws 114. In some embodiments, using attachment port 92 enables a user to pass one or more components and/or tools along a common guide wire, etc.

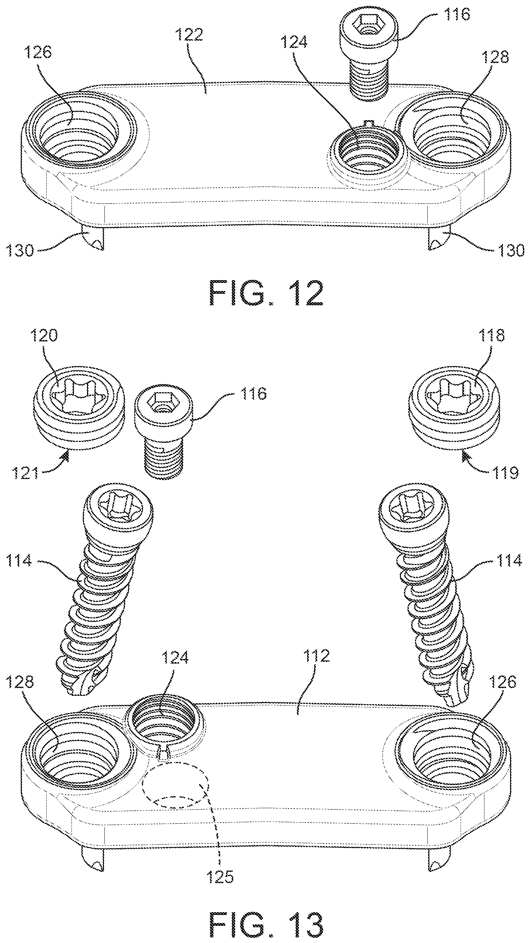

In some embodiments, plate 112 includes an access aperture 125 (see FIG. 13) that enables a tool such as tool 11 to access tool port 76 (see FIG. 4) while plate 112 is secured to implant 10. For example, plate 112 may be secured to expandable implant 10 prior to expansion of expandable implant 10 (e.g., to minimize migration of the implant). A tool may then access tool port 76 via access aperture 125 to adjust expandable implant 10 to a desired amount of expansion.

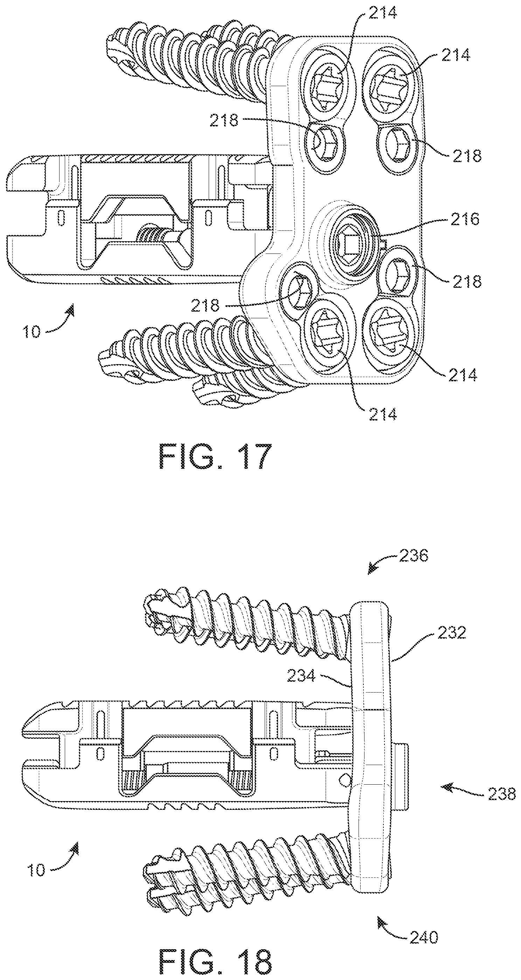

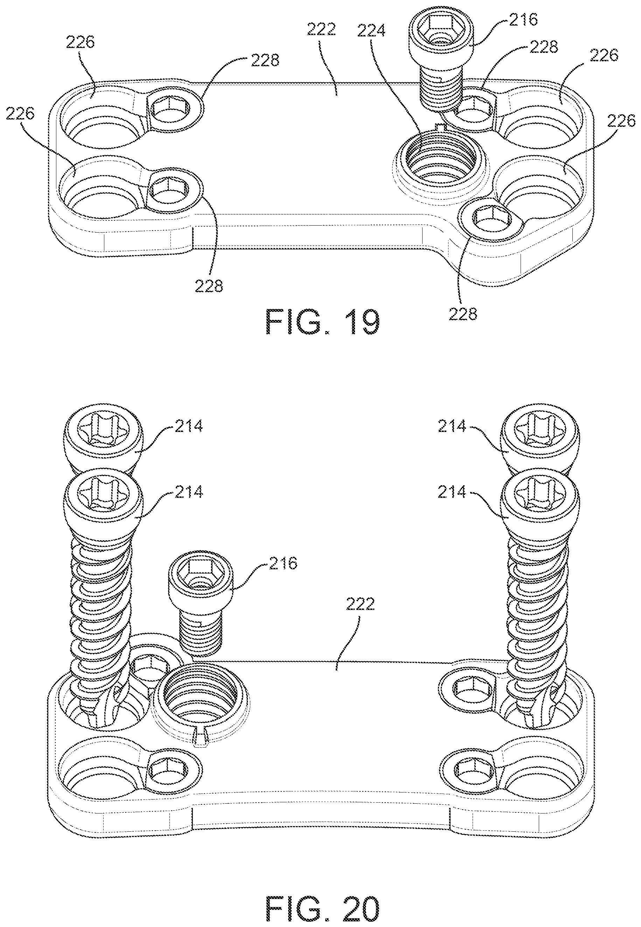

Referring now to FIGS. 17-23, an integrated implant 210 is shown according to one embodiment. Implant 210 includes implant 10, a plate 212, and bone screws 214. As discussed in greater detail below, once implant 10 is secured in a desired position, plate 212 may be secured (e.g., removably coupled) to implant 10, and bone screws 214 secured to adjacent portions of bone (e.g., adjacent portions of vertebral bone). Implant 210 may include any of the features described above with respect to implant 110, and all such combinations of features are to be understood to be within the scope of the present disclosure. Similarly, implant 110 may include any of the features discussed below with respect to implant 210. Implant 210 is generally similar to implant 110 except for the size and shape of the plate, the number of bone screws utilized, and the method of preventing backing out of the bone screws.

In one embodiment, plate 212 is secured to implant 10 using a coupling screw 216. Coupling screw 216 may in one embodiment threadingly engage attachment port 92 of implant 10. In this way, attachment port 92 may be used both to receive a tool used to initially position and manipulate implant 10, and to receive a coupling screw such as coupling screw 216 used to couple plate 212 with implant 10. Bone screws 214 are configured to engage adjacent portions of bone, and are held in place using retention fasteners 218. Retention fasteners 218 are configured to prevent bone screws 214 from backing out once installed. In one embodiment, retention fasteners include a head 218 having a flat portion configured to be adjacent the head of bone screw 214. Should bone screw 214 start to loosen, retention fastener 218 rotates to put increasing resistance upon the head of bone screw 214, thereby preventing bone screw 214 from backing out.

Plate 212 includes a body 222 having a top portion 236, a middle portion 238, and a bottom portion 240. Bone screw bores 226 are provided in top and bottom portions 236, 240 and receive bone screws 214. Retention fastener bores 228 are provided adjacent bone screw bores 226 and are configured to receive retention fasteners 218 (e.g., locking screws, etc.). The distance between and positions of bone screw bores 226 can be varied to suit a particular application. In one embodiment, bone screw bores 226 are positioned to engage corresponding upper and lower portions of bone once implant 10 is installed and expanded in a desired position and orientation.

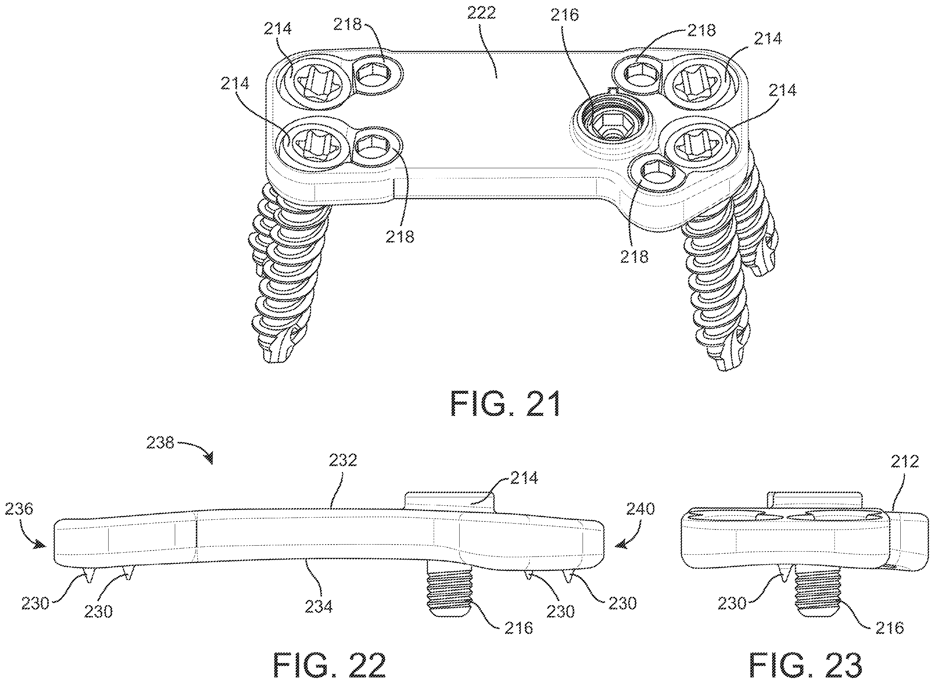

Plate 212 includes a front side 232 and a rear side 234. Bone screw bores 226 and coupling screw bore 224 extend from front side 232 to rear side 234. Projections 230 extend from rear side 234 and are configured to engage adjacent portions of bone. Projections 230 may assist in preventing rotation or other movement of plate 212 during installation and/or prior to installation of bone screws 214. Projections 230 may be shaped with a pointed, sharp, and/or knife-shaped end configured to facilitate pressing or otherwise forcing projections 230 into portions of bone. As shown in Figures 22-23, in one embodiment, four projections 230 are utilized with plate 212, and projections 230 are generally positioned along a line. In alternative embodiments, more or fewer projections 230 may be used, and the projections 230 may be positioned differently than shown in FIGS. 22-23. In one embodiment, plate 212 is a single, integral piece (e.g., a monolithic component). Providing an integral plate may provide increased stability relative to multi-piece plates.

In use, a user positions implant 10 in a desired position using attachment port 92 and a suitable tool. Implant 10 may be expanded to a desired height by manipulating control shaft 16. Once implant 10 is in a desired position and at a desired degree of expansion, plate 212 may be coupled with implant 10 by threadingly coupling the coupling screw 216 to attachment port 92. Plate 212 may be positioned such that projections 230 engage adjacent portions of bone. Bone screws 214 may be inserted through plate 212 and threadingly secured to adjacent portions of bone. Retention fasteners 218 may be installed to prevent backing out or loosening of bone screws 214. In some embodiments, using attachment port 92 enables a user to pass one or more components and/or tools along a common guide wire, etc.

In some embodiments, plate 212 includes an access aperture similar to access aperture 125 (see FIG. 13) that enables a tool such as tool 11 to access tool port 76 (see FIG. 4) while plate 212 is secured to implant 10. For example, plate 212 may be secured to expandable implant 10 prior to expansion of expandable implant 10 (e.g., to minimize migration of the implant). A tool may then access tool port 76 via access aperture 125 to adjust expandable implant 10 to a desired amount of expansion.

In some embodiments, implant assemblies 110, 210 are provided with multiple plates and/or with associated tooling such as tool 11. For example, an assembly (e.g., a kit, etc.) may include implant 10, a plurality of different plates (e.g., plate 112, 212, etc.), and a plurality of bone screws. Each plate may be configured to interchangeably couple with implant 10. Furthermore, a plurality of bone screws may be provided, and some or all of the bone screws may be utilized depending on the particular plate chosen for a specific application. For example, if a user chooses to use plate 112, two bone screws may be utilized, while if a user chooses to use plate 212, four bone screws may be utilized. Any of a number of combinations of implant 10, plates 110 and/or 210, and bone screws may be utilized to fit a particular application.

Referring back to the Figures generally, the various embodiments disclosed herein provide expandable implants including a base member, an adjustable member adjustably coupled to the base member and movable between a first, collapsed position, and a second, expanded position, and a control shaft rotatably received by the base member, where rotation of the control shaft cause relative movement of the adjustable member relative to the base member. At least one control member is received on the control shaft and by the control channel, and rotation of the control shaft causes the control member to translate along the control shaft and along the control channel.

In some embodiments, the adjustable member moves in a linear fashion relative to the base member. In other embodiments, the adjustable member moves in a non-linear fashion relative to the base member. In further embodiments, the adjustable member pivots (e.g., about a pivot axis or otherwise) relative to the base member. The pivot axis may be provided by a pivot pin extending through one or both of the adjustable member and the base member.

In some embodiments, a single control member and control channel are utilized. In other embodiments, multiple (e.g., 2) control members and control channels are utilized. In some embodiments, the multiple control channels are parallel and straight. In other embodiments, the control channels are non-parallel and straight (e.g., angled toward each other). In further embodiments, the control channels are non-parallel and non-straight such that the adjustable member moves in a non-linear fashion relative to the base member.

In some embodiments, the control shaft includes a control thread corresponding to each control member. As such, while in some embodiments the control shaft includes a single control thread, in other embodiments the control shaft includes multiple (e.g., first and second) control threads. In some embodiments, the control threads are like-threaded. In other embodiments, the control threads have different threads. For example, in some embodiments, a first control thread is opposite-handed from a second control thread. In further embodiments, a first control thread has a different pitch from a second control thread. In yet further embodiments, a first control thread is different handed and has a different pitch from a second control thread. In some embodiments, a first control thread has a larger diameter than a second control tread, enabling one control member to pass over one of the control threads in a non-threaded manner during assembly of the expandable implant.

In some embodiments, one or both of the adjustable member and the base member include projections / grooves to provide a gripping surface intended to facilitate gripping adjacent portions of bone. In further embodiments, one or both of the adjustable member and the base member include one or more apertures and/or cavities configured to promote bone growth in and around the adjustable member and the base member, and/or receive bone growth material. In some embodiments, the apertures extend from a top, bottom, and/or side surface of the adjustment member or the base member and to a central cavity of the implant.

According to any of the embodiments disclosed herein, multiple bone screws are used. In further embodiments, multiple bone screws are accessible and manipulatable by way of a front face of the implant assembly defined by the plate. The bone screws are generally positioned on opposite sides (e.g., top and bottom) of the expandable implant.

In some embodiments, a plate is secured to an adjustable implant after expansion of the expandable implant. In other embodiments, the plate is secured to the adjustable implant prior to expansion of the expandable implant. In further embodiments, one or more bone screws may be secured to adjacent portions of bone after securing the plate to the expandable implant but prior to expansion of the expandable implant. For example, prior to expansion of the expandable implant, the plate may be secured to a base member (e.g., a lower support member), and a bone screw may be secured through the portion of the plate that is on the side of and/or extends past/below the base member. In this way, migration of the expandable implant is minimized or prevented during expansion of the expandable implant. In one embodiment, the plate may be a buttress plate configured to attach to one or both of the base member and the adjustable member and further attach to an adjacent bone portion either before or after expansion or other height adjustment of the expandable implant.

As utilized herein, the terms "approximately," "about," "substantially," and similar terms are intended to have a broad meaning in harmony with the common and accepted usage by those of ordinary skill in the art to which the subject matter of this disclosure pertains. It should be understood by those of skill in the art who review this disclosure that these terms are intended to allow a description of certain features described and claimed without restricting the scope of these features to the precise numerical ranges provided. Accordingly, these terms should be interpreted as indicating that insubstantial or inconsequential modifications or alterations of the subject matter described and claimed are considered to be within the scope of the disclosure as recited in the appended claims.

It should be noted that the term "exemplary" and variations thereof, as used herein to describe various embodiments, are intended to indicate that such embodiments are possible examples, representations, or illustrations of possible embodiments (and such terms are not intended to connote that such embodiments are necessarily extraordinary or superlative examples).

The term "coupled" and variations thereof, as used herein, means the joining of two members directly or indirectly to one another. Such joining may be stationary (e.g., permanent or fixed) or moveable (e.g., removable or releasable). Such joining may be achieved with the two members coupled directly to each other, with the two members coupled to each other using a separate intervening member and any additional intermediate members coupled with one another, or with the two members coupled to each other using an intervening member that is integrally formed as a single unitary body with one of the two members. If "coupled" or variations thereof are modified by an additional term (e.g., directly coupled), the generic definition of "coupled" provided above is modified by the plain language meaning of the additional term (e.g., "directly coupled" means the joining of two members without any separate intervening member), resulting in a narrower definition than the generic definition of "coupled" provided above. Such coupling may be mechanical, electrical, or fluidic.

The term "or," as used herein, is used in its inclusive sense (and not in its exclusive sense) so that when used to connect a list of elements, the term "or" means one, some, or all of the elements in the list. Conjunctive language such as the phrase "at least one of X, Y, and Z," unless specifically stated otherwise, is understood to convey that an element may be either X, Y, Z; X and Y; X and Z; Y and Z; or X, Y, and Z (i.e., any combination of X, Y, and Z). Thus, such conjunctive language is not generally intended to imply that certain embodiments require at least one of X, at least one of Y, and at least one of Z to each be present, unless otherwise indicated.

References herein to the positions of elements (e.g., "top," "bottom," "above," "below") are merely used to describe the orientation of various elements in the FIGURES. It should be noted that the orientation of various elements may differ according to other exemplary embodiments, and that such variations are intended to be encompassed by the present disclosure.

It is important to note that the construction and arrangement of the elements of the various implants and implant components as shown in the exemplary embodiments are illustrative only. Although a few embodiments have been described in detail in this disclosure, those skilled in the art who review this disclosure will readily appreciate that many modifications are possible (e.g., variations in sizes, dimensions, structures, shapes and proportions of the various elements, values of parameters, mounting arrangements, materials, colors, orientations, etc.) without materially departing from the novel teachings and advantages of the subject matter recited in the various embodiments. Accordingly, all such modifications are intended to be included within the scope of the present disclosure.

## Claims

1. An implant assembly, comprising:
an expandable implant (10), the expandable implant comprising a base member (12) and an adjustable member (14) movable relative to the base member such that the expandable implant is movable between a collapsed configuration and an expanded configuration, wherein the base member comprises an attachment port (92);
a plate (112, 212) configured to be coupled to the base member;
a coupling fastener (116, 216) configured to couple the plate (112, 212) to the base member (12) such that the plate (112, 212) is fixed relative to the base member (12) while the adjustable member (14) is movable relative to the base member (12) between the collapsed configuration and the expanded configuration; and
at least one bone screw (114, 214) configured to extend through the plate (112, 212) and into an adjacent portion of bone;
wherein the attachment port (92) is configured to receive an installation tool (11) during positioning of the expandable implant and to receive the coupling fastener (116, 216) to couple the plate (112, 212) to the base member (12).

2. The implant assembly of claim 1, wherein the expandable implant comprises a control assembly configured to control movement of the expandable implant (10) between the collapsed configuration and the expanded configuration

3. The implant assembly of claim 2, wherein the control assembly comprises first and second control members (20, 22) received on a control shaft (16).

4. The implant assembly of claim 3, wherein the adjustable member (14) comprises a first control channel (68) configured to receive the first control member (20) and a second control channel (70) configured to receive the second control member (22).

5. The implant assembly of claim 4, wherein the first control member (20) includes a first projection (72) configured to engage the first control channel (68) on the adjustable member (14) to prevent rotation of the first control member (20) within the first control channel (68).

6. The implant assembly of claim 5, wherein the second control member (22) includes a second projection (72) configured to engage the second control channel (70) on the adjustable member (14) to prevent rotation of the second control member (22) within the second control channel (70).

7. The implant assembly of claim 3, wherein the control shaft (16) comprises a first control thread (78) having a first diameter and configured to threadingly receive the first control member (20).

8. The implant assembly of claim 7, wherein the control shaft (16) comprises a second control thread (80) having a second diameter smaller than the first diameter and configured to threadingly receive the second control member (22).

9. The implant assembly of claim 3, wherein each of the first and second control members (20, 22) are configured to provide a first force to the adjustable member (14) to move the adjustable member (14) toward the expanded configuration.

10. The implant assembly of claim 9, wherein each of the first and second control members (20, 22) are configured to provide a second force to the adjustable member (14) to move the adjustable member (14) toward the collapsed configuration.

11. The implant assembly of claim 1, wherein the plate (112, 212) comprises a front side (132, 232) and a rear side (134, 234), wherein the rear side (134, 234) faces the expandable implant (10) and a plurality of projections (130, 230) extend outward from the rear side (134, 234) and are configured to engage adjacent portions of bone.

12. The implant assembly of claim 1, wherein the attachment port (92) is provided on a front face on the expandable implant (10).

13. The implant assembly of claim 1, wherein the base member (12) further comprises first and second anti-rotation ports (94, 96) provided on opposite sides of the attachment port (92).

14. The implant assembly of claim 13, wherein the first and second anti-rotation ports (94, 96) are configured to receive portions (13) of the installation tool (11) to prevent rotation of the expandable implant (10).

15. The implant assembly of claim 1, wherein the at least one bone screw (114, 214) comprises a first bone screw (114, 214) and a second bone screw (114, 214); and
wherein the plate (112, 212) comprises a first bone screw bore (126, 226) positioned on a first end (136, 236) of the plate (112) when the expandable implant (10) is coupled to the plate (112, 212), and a second bone screw bore (128, 226) positioned on a second end (140, 240) of the plate (112, 212) opposite the first end (136, 236) when the expandable implant (10) is coupled to the plate (112, 212).

## Patentansprüche

1. Implantatanordnung, die Folgendes umfasst:
ein ausfahrbares Implantat (10), wobei das ausfahrbare Implantat ein Basiselement (12) und ein einstellbares Element (14) umfasst, das relativ zu dem Basiselement beweglich ist, so dass das ausfahrbare Implantat zwischen einer eingefahrenen Konfiguration und einer ausgefahrenen Konfiguration beweglich ist, wobei das Basiselement eine Befestigungsöffnung (92) umfasst;
eine Platte (112, 212), die so gestaltet ist, dass sie mit dem Basiselement verbunden werden kann;
ein Kopplungsbefestigungselement (116, 216), das so konfiguriert ist, dass es die Platte (112, 212) mit dem Basiselement (12) koppelt, so dass die Platte (112, 212) relativ zu dem Basiselement (12) fixiert ist, während das einstellbare Element (14) relativ zu dem Basiselement (12) zwischen der eingefahrenen Konfiguration und der ausgefahrenen Konfiguration beweglich ist; und
mindestens eine Knochenschraube (114, 214), die so gestaltet ist, dass sie sich durch die Platte (112, 212) und in einen angrenzenden Knochenabschnitt erstreckt;
wobei die Befestigungsöffnung (92) so konfiguriert ist, dass sie während der Positionierung des ausfahrbaren Implantats ein Installationswerkzeug (11) aufnimmt und das Kopplungsbefestigungselement (116, 216) aufnimmt, um die Platte (112, 212) mit dem Basiselement (12) zu koppeln.

2. Implantatanordnung nach Anspruch 1, wobei das ausfahrbare Implantat eine Steueranordnung umfasst, die so konfiguriert ist, dass sie die Bewegung des ausfahrbaren Implantats (10) zwischen der eingefahrenen Konfiguration und der ausgefahrenen Konfiguration steuert.

3. Implantatanordnung nach Anspruch 2, wobei die Steueranordnung ein erstes und ein zweites Steuerelement (20, 22) umfasst, die auf einer Steuerwelle (16) aufgenommen werden.

4. Implantatanordnung nach Anspruch 3, wobei das einstellbare Element (14) einen ersten Steuerkanal (68), der zur Aufnahme des ersten Steuerelements (20) konfiguriert ist, und einen zweiten Steuerkanal (70), der zur Aufnahme des zweiten Steuerelements (22) konfiguriert ist, umfasst.

5. Implantatanordnung nach Anspruch 4, wobei das erste Steuerelement (20) einen ersten Vorsprung (72) aufweist, der so konfiguriert ist, dass er in den ersten Steuerkanal (68) auf dem einstellbaren Element (14) eingreift, um eine Drehung des ersten Steuerelements (20) innerhalb des ersten Steuerkanals (68) zu verhindern.

6. Implantatanordnung nach Anspruch 5, wobei das zweite Steuerelement (22) einen zweiten Vorsprung (72) aufweist, der so konfiguriert ist, dass er in den zweiten Steuerkanal (70) auf dem einstellbaren Element (14) eingreift, um eine Drehung des zweiten Steuerelements (22) innerhalb des zweiten Steuerkanals (70) zu verhindern.

7. Implantatanordnung nach Anspruch 3, wobei die Steuerwelle (16) ein erstes Steuergewinde (78) umfasst, mit einem ersten Durchmesser, und das so konfiguriert ist, dass es das erste Steuerelement (20) gewindemäßig aufnimmt.

8. Implantatanordnung nach Anspruch 7, wobei die Steuerwelle (16) ein zweites Steuergewinde (80) umfasst, mit einem zweiten Durchmesser, der kleiner als der erste Durchmesser ist, und das so konfiguriert ist, dass es das zweite Steuerelement (22) gewindemäßig aufnimmt.

9. Implantatanordnung nach Anspruch 3, wobei jedes der ersten und zweiten Steuerelemente (20, 22) so konfiguriert ist, dass es eine erste Kraft auf das einstellbare Element (14) ausübt, um das einstellbare Element (14) in Richtung der ausgefahrenen Konfiguration zu bewegen.

10. Implantatanordnung nach Anspruch 9, wobei jedes der ersten und zweiten Steuerelemente (20, 22) so konfiguriert ist, dass es eine zweite Kraft auf das einstellbare Element (14) ausübt, um das einstellbare Element (14) in Richtung der eingefahrenen Konfiguration zu bewegen.

11. Implantatanordnung nach Anspruch 1, wobei die Platte (112, 212) eine Vorderseite (132, 232) und eine Rückseite (134, 234) umfasst, wobei die Rückseite (134, 234) dem ausfahrbaren Implantat (10) zugewandt ist und mehrere Vorsprünge (130, 230) sich von der Rückseite (134, 234) nach außen erstrecken und so konfiguriert sind, dass sie in benachbarte Knochenabschnitte eingreifen.

12. Implantatanordnung nach Anspruch 1, wobei die Befestigungsöffnung (92) an einer Vorderseite des ausfahrbaren Implantats (10) vorgesehen ist.

13. Implantatanordnung nach Anspruch 1, wobei das Basiselement (12) ferner eine erste und eine zweite Antirotationsöffnung (94, 96) umfasst, die auf gegenüberliegenden Seiten der Befestigungsöffnung (92) vorgesehen sind.

14. Implantatanordnung nach Anspruch 13, wobei die erste und die zweite Antirotationsöffnung (94, 96) so konfiguriert sind, dass sie Teile (13) des Installationswerkzeugs (11) aufnehmen, um eine Drehung des ausfahrbaren Implantats (10) zu verhindern.

15. Implantatanordnung nach Anspruch 1, wobei die mindestens eine Knochenschraube (114, 214) eine erste Knochenschraube (114, 214) und eine zweite Knochenschraube (114, 214) umfasst; und
wobei die Platte (112, 212) eine erste Knochenschraubenbohrung (126, 226) umfasst, die an einem ersten Ende (136, 236) der Platte (112) positioniert ist, wenn das ausfahrbare Implantat (10) mit der Platte (112, 212) gekuppelt ist, und eine zweite Knochenschraubenbohrung (128, 226), die an einem zweiten Ende (140, 240) der Platte (112, 212) gegenüber dem ersten Ende (136, 236) positioniert ist, wenn das ausfahrbare Implantat (10) mit der Platte (112, 212) gekuppelt ist.

## Revendications

1. Ensemble d'implant comprenant :
un implant expansible (10), l'implant expansible comprenant un élément de base (12) et un élément réglable (14) mobile par rapport à l'élément de base de sorte que l'implant expansible est mobile entre une configuration repliée et une configuration expansée, dans lequel l'élément de base comprend un orifice de fixation (92) ;
une plaque (112, 212) configurée pour être couplée à l'élément de base ;
une fixation de couplage (116, 216) configurée pour coupler la plaque (112, 212) à l'élément de base (12), de sorte que la plaque (112, 212) est fixe par rapport à l'élément de base (12), alors que l'élément réglable (14) est mobile par rapport à l'élément de base (12) entre la configuration repliée et la configuration expansée ; et
au moins une vis à os (114, 214) configurée pour s'étendre à travers la plaque (112, 212) et dans une partie d'os adjacente ;
dans lequel l'orifice de fixation (92) est configuré pour recevoir un outil d'installation (11) pendant le positionnement de l'implant expansible et pour recevoir la fixation de couplage (116, 216) afin de coupler la plaque (112, 212) à l'élément de base (12).

2. Ensemble d'implant selon la revendication 1, dans lequel l'implant expansible comprend un ensemble de commande configuré pour commander le déplacement de l'implant expansible (10) entre la configuration repliée et la configuration expansée.

3. Ensemble d'implant selon la revendication 2, dans lequel l'ensemble de commande comprend des premier et deuxième éléments de commande (20, 22) reçus sur un arbre de commande (16).

4. Ensemble d'implant selon la revendication 3, dans lequel l'élément réglable (14) comprend un premier canal de commande (68) configuré pour recevoir le premier élément de commande (20) et un deuxième canal de commande (70) configuré pour recevoir le deuxième élément de commande (22).

5. Ensemble d'implant selon la revendication 4, dans lequel le premier élément de commande (20) comprend une première saillie (72) configurée pour mettre en prise le premier canal de commande (68) sur l'élément réglable (14) afin d'empêcher la rotation du premier élément de commande (20) à l'intérieur du premier canal de commande (68).

6. Ensemble d'implant selon la revendication 5, dans lequel le deuxième élément de commande (22) comprend une deuxième saillie (72) configurée pour mettre en prise le deuxième canal de commande (70) sur l'élément réglable (14) afin d'empêcher la rotation du deuxième élément de commande (22) à l'intérieur du deuxième canal de commande (70).

7. Ensemble d'implant selon la revendication 3, dans lequel l'arbre de commande (16) comprend un premier filetage de commande (78) ayant un premier diamètre et configuré pour recevoir, par filetage, le premier élément de commande (20).

8. Ensemble d'implant selon la revendication 7, dans lequel l'arbre de commande (16) comprend un deuxième filetage de commande (80) ayant un deuxième diamètre inférieur au premier diamètre et configuré pour recevoir, par filetage, le deuxième élément de commande (22).

9. Ensemble d'implant selon la revendication 3, dans lequel chacun des premier et deuxième éléments de commande (20, 22) est configuré pour fournir une première force à l'élément réglable (14) pour déplacer l'élément réglable (14) vers la configuration expansée.

10. Ensemble d'implant selon la revendication 9, dans lequel chacun des premier et deuxième éléments de commande (20, 22) est configuré pour fournir une deuxième force à l'élément réglable (14) pour déplacer l'élément réglable (14) vers la configuration repliée.

11. Ensemble d'implant selon la revendication 1, dans lequel la plaque (112, 212) comprend un côté avant (132, 232) et un côté arrière (134, 234), dans lequel le côté arrière (134, 234) fait face à l'implant expansible (10) et une pluralité de saillies (130, 230) s'étendent vers l'extérieur à partir du côté arrière (134, 234) et sont configurées pour mettre en prise des parties d'os adjacentes.

12. Ensemble d'implant selon la revendication 1, dans lequel l'orifice de fixation (92) est prévu sur une face avant sur l'implant expansible (10).

13. Ensemble d'implant selon la revendication 1, dans lequel l'élément de base (12) comprend en outre des premier et deuxième orifices anti-rotation (94, 96) prévus sur des côtés opposés de l'orifice de fixation (92).

14. Ensemble d'implant selon la revendication 13, dans lequel les premier et deuxième orifices anti-rotation (94, 96) sont configurés pour recevoir des parties (13) de l'outil d'installation (11) pour empêcher la rotation de l'implant expansible (10).

15. Ensemble d'implant selon la revendication 1, dans lequel la au moins une vis à os (114, 214) comprend une première vis à os (114, 214) et une deuxième vis à os (114, 214) ; et
dans lequel la plaque (112, 212) comprend un premier alésage de vis à os (126, 226) positionné sur une première extrémité (136, 236) de la plaque (112) lorsque l'implant expansible (10) est couplé à la plaque (112, 212) et un deuxième alésage de vis à os (128, 226) positionné sur une deuxième extrémité (140, 240) de la plaque (112, 212) opposée à la première extrémité (136, 236), lorsque l'implant expansible (10) est couplé à la plaque (112, 212).
